(19) Europäisches Patentamt
European Patent Office
Office européen des brevets

(11) **EP 1 223 798 B1**

(12) **FASCICULE DE BREVET EUROPEEN**

(45) Date de publication et mention
de la délivrance du brevet:
**08.09.2004 Bulletin 2004/37**

(51) Int Cl.[7]: **A01H 1/04**, A01H 1/06

(21) Numéro de dépôt: **00974579.5**

(86) Numéro de dépôt international:
**PCT/FR2000/002997**

(22) Date de dépôt: **27.10.2000**

(87) Numéro de publication internationale:
**WO 2001/030133 (03.05.2001 Gazette 2001/18)**

(54) **PROCEDE DE PRODUCTION D'UNE BANQUE OU COLLECTION DE MUTANTS ET SES APPLICATIONS**

VERFAHREN ZUM ERZEUGEN EINES BESTANDS ODER EINER SAMMLUNG VON MUTANTEN UND DESSEN VERWENDUNGEN

METHOD FOR PRODUCING A BANK OR COLLECTION OF MUTANTS AND APPLICATIONS THEREOF

(84) Etats contractants désignés:
**AT BE CH CY DE DK ES FI FR GB GR IE IT LI LU MC NL PT SE**

(30) Priorité: **28.10.1999 FR 9913515**

(43) Date de publication de la demande:
**24.07.2002 Bulletin 2002/30**

(73) Titulaire: **COMMISSARIAT A L'ENERGIE ATOMIQUE**
**75015 Paris Cédex 15 (FR)**

(72) Inventeurs:
• **TISSIER, Alain**
**F-84120 Pertuis (FR)**
• **MONTANE, Marie-Hélène**
**13100 Aix-en-provence (FR)**

(74) Mandataire: **Orès, Bernard et al**
**Cabinet ORES**
**36,rue de St Pétersbourg**
**75008 Paris (FR)**

(56) Documents cités:
• **VIZIR ET AL.: "Genetics of Gamma-Irradiation-Induced mutations in Arabidopsis thaliana: large chromosal deletions can be rescued through the fertilization of diploid eggs" JOURNAL OF HEREDITY, vol. 90, mai 1999 (1999-05), pages 412-417, XP000925237**
• **PONCE ET AL.: "High-throughput genetic mapping in Arabidopsis thaliana" MOLECULAR AND GENERAL GENETICS, vol. 261, mars 1999 (1999-03), pages 408-415, XP000929112**
• **DATABASE BIOSIS [en ligne] BIOSCIENCES INFORMATION SERVICE, PHILADELPHIA, PA, US; AN: PREV199293099919, 1992 REITER ET AL.: "Global and local genome mapping in Arabidopsis thaliana by using recombinant inbred lines and random amplified polymorfic DNAs" XP002145886**

## Description

La présente invention est relative à un procédé de production d'une banque ou collection de mutants aptes à permettre l'étude de la fonction de chaque gène, fragment d'ADN ou séquence définie d'une espèce de plante donnée et à ses applications : banques de mutants et procédé de sélection d'un mutant d'un gène.

Le séquençage systématique des génomes d'espèces modèles (homme, bactéries, levure, *Caenorhabditis elegans, Arabidopsis thaliana,* riz) fournit une quantité d'informations qui, au-delà de l'aspect fondamental, permet l'identification chez l'Homme et certains animaux, de gènes impliqués dans des pathologies et peut déboucher sur de nouvelles thérapies, tandis que chez les végétaux, la caractérisation de gènes responsables de traits agronomiques permettra d'accélérer l'amélioration des espèces cultivées. Avec l'analyse globale de l'expression du génome (transcriptome et protéome) l'autre axe essentiel à développer en biologie est l'analyse fonctionnelle systématique du génome. Il s'agit dans ce cas, de disposer pour chaque gène d'allèles mutants (nuls ou partiels), de façon à en évaluer le rôle dans le développement et la physiologie de l'organisme.

Les outils d'analyse fonctionnelle globale du génome actuellement utilisés chez les modèles d'étude végétaux ou animaux reposent essentiellement :

- sur la mutagenèse insertionnelle : les mutagènes insertionnels (transposons ou T-DNA chez les plantes) peuvent être facilement détectés par PCR en prenant une amorce de l'agent insertionnel et une amorce spécifique du gène (Martienssen, 1998). Cette approche a été développée entre autres chez la drosophile (Kaiser et al., 1990), la levure (Smith et al., 1995) le nématode *Caenorhabditis elegans* (Zwaal et al., 1993), et *Arabidopsis thaliana* (Azpiroz-Leehan et al., 1997; Bouchez et al., 1998). Toutefois, les banques de mutants insertionnels sont difficiles à constituer et lourdes à mettre en place ; elles ont en outre l'inconvénient d'être soumises aux contraintes de confinement des organismes génétiquement modifiés ; elles ne sont en outre pas facilement transférables à des espèces cultivées,

- sur le remplacement ciblé par recombinaison homologue (knock-out), qui n'est pas praticable en routine chez les végétaux supérieurs et

- sur les réarrangements suffisamment importants comme des délétions pour pouvoir être détectés par PCR. Cette approche a été utilisée chez le poisson zèbre avec les rayons gamma (Fritz et al., 1996) et chez le nématode par mutagenèse chimique (Jansen et al., 1997). Chacune de ces deux espèces présente des particularités qui y ont rendu possible ce type d'approche. En effet :

  - chez le poisson zèbre d'abord, il a été tiré parti de la possibilité de produire des organismes haploïdes par parthénogenèse. Ainsi, les délétions chez un organisme haploïde peuvent être mises en évidence par l'absence de produit de PCR, puisque l'allèle sauvage est absent, à condition toutefois que l'ADN soit analysé individu par individu. Cela est peu efficace lorsque l'on doit analyser des milliers de mutants.
  - le nématode *Caenorhabditis elegans* est un organisme hermaphrodite dont on peut facilement cultiver des dizaines de milliers d'individus. C'est par une mutagenèse chimique produisant des délétions à faible fréquence (15 %) et sur une collection de plus de 100 000 individus que les études sur cet animal ont été menées. Les délétions sont repérées par l'apparition de produits PCR de taille inférieure à celle de l'allèle sauvage. L'ADN des mutants est extrait non pas individuellement mais en lots de taille variable, de manière à pouvoir minimiser le nombre de PCR à effectuer. Ainsi, les premières réactions sont faites sur des lots de 1000 individus, puis sur des sous-lots de 10, et enfin sur des individus.

De telles méthodes ne sont donc pas utilisables pour des organismes ne présentant pas les particularités précitées.

Les Inventeurs ont maintenant mis au point un procédé qui répond mieux aux besoins de la pratique, notamment en ce qu'il met en oeuvre des rayonnements ionisants et en ce qu'il est adapté à l'étude fonctionnelle de la totalité du génome d'une plante.

Les rayonnements ionisants (RI) (rayons X, gamma, neutrons rapides, ions lourds) sont de puissants mutagènes qui induisent majoritairement des réarrangements importants, essentiellement des délétions (> 75 %). Ils ont été utilisés chez un certain nombre d'organismes animaux dont la drosophile (Kelley et al., 1985), le poisson medaka (Kubota et al., 1995), le poisson zèbre (Chakrabarti et al 1983), la souris (Cattanach et al., 1993 ; Turker et al., 1997), les cellules de hamster ou humaines (Schwartz et al., 1991 ; Giver et al., 1995), et également chez de nombreux végétaux, dont le maïs (Fujii, 1978), le riz (Purushothaman, 1969), la légumineuse *Medicago truncatula* (Sagan et al., 1995) et la plante modèle *Arabidopsis thaliana* (Redei, 1974 ; Shirley et al., 1992 ; Sun et al., 1992).

La nature moléculaire des mutations induites par les RI a été analysée chez certains de ces organismes essentiellement par l'étude des mutants obtenus pour des gènes déjà connus (Bruggemann et al., 1996 ; Fuscoe et al., 1992). Les types de mutations observés sont les suivants : délétions, translocations, inversions, insertions et mutations ponctuelles. Ces types ne sont pas nécessairement exclusifs. Par exemple, une délétion peut être accompagnée d'une insertion, ou d'une inversion (Shirley et al., 1992).

Quel que soit l'organisme eucaryote étudié, les délétions sont le type dominant avec des fréquences autour de 70 % (Fuscoe et al., 1992 ; Bruggeman et al., 1996 ; Cecchini et al., 1998). La taille des délétions est variable mais les délétions totales du gène représentent la majorité (environ 50 %) des mutations et les délétions partielles de 10 à 25 %.

Le gène *HY4 d'Arabidopsis thaliana* (Bruggeman et al., 1996), par exemple, couvre environ 4 kb. Sur les 20 mutants *hy4* isolés, 11 (55 %) présentent une délétion totale excédant 8 kb, 1 (5 %) une délétion totale inférieure à 8 kb, 3 (15 %) des délétions avec une borne dans le gène et l'autre à l'extérieur du gène, 2 (10 %) des délétions aux bornes contenues dans le gène. Le reste, 3 (15 %), ne présente pas d'altérations détectables par hybridation génomique.

Toutefois, l'utilisation des rayonnements ionisants, telle que décrite à ce jour, s'est limitée à la caractérisation de mutations pour des gènes connus (Bruggeman et al., 1996 ; Cecchini et al. 1998) ou au clonage par hybridation soustractive de gènes mutés (Sun et al., 1992).

C'est pourquoi les Inventeurs se sont donnés pour but de fournir un procédé pour l'établissement de collections optimales de mutants de plantes induits par les radiations ionisantes, afin de permettre l'étude de la fonction de chaque gène ou fragment d'ADN et ce, quelle que soit l'espèce considérée.

La présente invention a pour objet un procédé de production d'une collection ou banque de mutants pour l'étude de la fonction de chaque gène d'une plante A sélectionnée, caractérisé en ce qu'il comprend :

**(a)** une estimation théorique du nombre n de mutations, induites par les rayonnements ionisants, nécessaires et suffisantes pour que chaque gène du génome de ladite plante A soit affecté par au moins l'une d'entre elles, à partir de la formule :

$$P_n = 1 - ((N-1)/N)^n \qquad \text{(formule 1)},$$

dans laquelle:

$P_n$ représente la probabilité qu'un gène donné soit muté et
N représente le nombre total estimé de gènes d'un organisme donné
On a $q_n = 1 - p_n$. Pour une mutation donnée, $q_1 = (N-1)/N$.

Si l'on suppose que les mutations sont indépendantes les unes des autres, pour n mutations, on a donc $q_n = ((N-1)/N)^n$.

Les valeurs pour des génomes fictifs de 20 000, 25 000 et 30 000 gènes sont présentées au Tableau I.

Tableau I :

| Nombre de Mutations n Nécessaires pour Obtenir un Mutant Donné avec une Probabilité pn[a] | | | |
|---|---|---|---|
| $P_n$ | Nombre de Gènes N | | |
| | 20000 | 25000 | 30000 |
| 0,90 | 46050 | 57563 | 69076 |
| 0,95 | **59913** | 74892 | 89870 |
| 0,99 | 92101 | 115127 | 138153 |

[a] $p_n = 1 - ((N-1)/N)^n$

**(b)** l'irradiation de graines sauvages par une dose d'irradiation x, choisie de manière à obtenir une densité de mutation par plante m et une fréquence de mutation dans un gène $y_x$ souhaitées, la relation entre *x, m* et $y_x$ étant représentée par les formules 2 et 3 suivantes :

$$m = n/y_x \qquad \text{(formule 2)},$$

dans laquelle n a la même signification qu'à la formule 1 et m est le nombre de mutations par plante ;

$$y_x = ax + f_s \qquad \text{(formule 3),}$$

dans laquelle :

$\underline{a}$ est le facteur de mutagenèse et $f_s$ est la fréquence de mutation spontanée et l'obtention de graines mutées de génération M1.

Les graines mutées et les plantes qui en dérivent sont désignées génération M1.

La densité de mutation désirée par plante dépend donc de la dose d'irradiation appliquée. Le choix de cette dose, puisqu'il détermine le nombre de mutations par plante, détermine également la complexité (= nombre total d'individus) de la banque et reflète l'efficacité de mutagenèse par le rayonnement utilisé.

La relation entre *x* et $y_x$ est considérée comme linéaire et reflète l'efficacité de mutagenèse par le rayonnement utilisé.

Pour les valeurs de $y_x$ considérées, $f_s$ est considérée comme négligeable. La valeur de $\underline{a}$ est déterminée expérimentalement.

La taille de la banque, correspondant au nombre total de mutations n(t), doit être telle que chaque région du génome soit mutée au moins une fois. On a donc n(t)=R.**n** où R exprime la redondance de la collection et est supérieur ou égal à 1 et **n** a la même signification que ci-dessus. Par redondance, on entend le nombre moyen de mutations présentes dans la collection pour un gène donné. En effet, la caractérisation d'un phénotype mutant a d'autant plus de valeur qu'elle est confirmée par des allèles indépendants. Une collection représentant au moins une fois et de préférence 5 à 10 fois le génome ($5 \leq R \leq 10$) est donc réaliste pour disposer d'une diversité d'allèles suffisante.

**(c)** l'obtention des plantes de génération M1, issues des graines mutées (irradiées) M1 obtenues en **(b)**, par germination et culture.

Les graines et plantes des générations suivantes dérivées par autofécondation, dans le cas *d'Arabidopsis,* par exemple, sont appelées M2, M3, ..., Mn.

Les graines ou plantes issues d'un même parent sont groupées sous le terme de famille et sont décrites comme soeurs.

**(d₁)** la récolte des graines soeurs M2 pour chaque plante M1, de préférence mise en pot individuellement, l'extraction de l'ADN à partir de plantules soeurs M2 cultivées en milieu solide (terreau ou milieu gélosé) ou en milieu liquide, et le regroupement dudit ADN en lots correspondant à plusieurs familles M2 qui servent de matrice pour la recherche des réarrangements génétiques ; la collection de mutants est donc représentée à la fois par les graines des familles M2 et par l'ADN extrait de ces familles ; et

**(e₁)** la détection des familles M2 portant une mutation dans un gène donné, effectuée sur l'ADN obtenu en (d₁), par un criblage reposant sur des techniques de PCR, aptes à détecter des réarrangements génétiques, réalisé sur des lots d'ADN de taille décroissante (recherche pyramidale desdits réarrangements) ; la taille et le type de réarrangement détectés dépendent de la technique utilisée et de la position des amorces. Par conséquent, il est possible de détecter des réarrangements affectant une partie ou la totalité d'un gène ou de plusieurs gènes.

Conformément à ce mode de mise en oeuvre du procédé, pour réduire le nombre de PCR à effectuer à l'étape (e₁) de criblage des réarrangements génétiques, les ADN des lots de graines M2 issues de plantes M1 sont eux-mêmes regroupés en super-lots.

En variante, ledit procédé comprend :

**(a)** une estimation théorique du nombre n de mutations, induites par les rayonnements ionisants, nécessaires et suffisantes pour que chaque gène du génome de ladite plante A soit affecté par au moins l'une d'entre elles, à partir de la formule :

$$p_n = 1 - ((N-1)/N)^n \qquad \text{(formule 1),}$$

dans laquelle :

$p_n$ représente la probabilité qu'un gène donné soit muté et,
N représente le nombre total estimé de gènes d'un organisme donné,

**(b)** l'irradiation de graines sauvages, par une dose d'irradiation *x*, choisie, de manière à obtenir une densité de mutation par plante *m* et une fréquence de mutation dans un gène $y_x$ souhaitées, la relation entre *x, m* et $y_x$ étant représentée par les formules 2 et 3 suivantes :

$$m = n/y_x \qquad \text{(formule 2),}$$

dans laquelle **n** a la même signification qu'à la formule 1 et *m* est le nombre de mutations par plante ;

$$y_x = ax + f_s \qquad \text{(formule 3),}$$

dans laquelle :

$\underline{a}$ est le facteur de mutagenèse et $f_s$ est la fréquence de mutation spontanée et l'obtention de graines mutées de génération M1 mutées.

**(c)** l'obtention des plantes de génération M1, issues des graines mutées M1 obtenues en **(b)**, par germination et culture.
**(d₂)** la récolte des graines soeurs M2 pour chaque plante M1, l'obtention des plantes de génération M2 par germination et culture, et l'extraction de l'ADN de plantes M2 individuelles ; l'ADN d'un échantillon représentatif de plantes M2, est extrait et analysé par plante ; et
**(e₂)** la détection et la localisation des réarrangements portés par ces plantes par marquage et hybridation de chaque ADN obtenu en **(d₂)** avec une puce de génotypage à oligonucléotides.

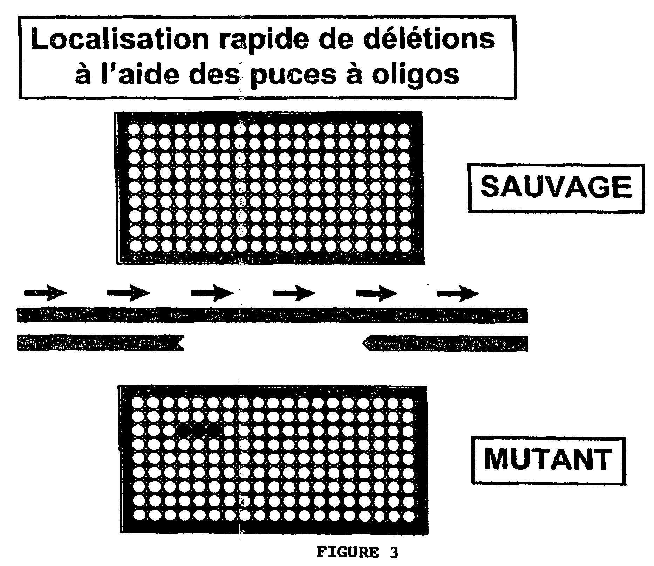

Conformément à l'invention, ladite étape **(e₂)** de détection est mise en oeuvre sur des puces à ADN : l'utilisation de puces à ADN sur des mutants induits par les rayonnements ionisants facilite considérablement la mise en oeuvre du procédé selon l'invention. En effet, la majorité des mutations induites par les rayonnements ionisants étant des délétions, l'hybridation de l'ADN d'un mutant de génération Mi (i≥2) sur une puce à ADN couvrant l'ensemble du génome, conduit à une perte ou à une diminution du signal par rapport à l'ADN sauvage sur les domaines de la puce correspondant à la délétion. Ainsi une seule hybridation permet de déterminer avec précision la localisation des mutations présentes et détectées chez le mutant en question. Ainsi l'application de ce procédé à un ensemble de plantes de génération Mi (i≥2) appartenant à la collection permettra de constituer une base de données contenant les informations pertinentes concernant le nombre, la localisation et éventuellement l'étendue, des mutations présentes et détectées chez les plantes dudit ensemble. La recherche de plantes de la collection portant une ou plusieurs mutations dans un gène donné pourra donc se faire par consultation de ladite base de données. (figure 3).
En variante, l'étape **(e₂)** de criblage des réarrangements génétiques est réalisée par une technique de PCR quantitative, de préférence par PCR quantitative en temps réel.
A titre d'exemple et de façon non limitative on peut citer le protocole de PCR quantitative en temps réel décrit par Chiang et al., 1999. La PCR quantitative en temps réel permet avantageusement de distinguer un individu hétérozygote pour une délétion, d'un individu homozygote sauvage, et *a fortiori* un individu homozygote pour la délétion d'un homozygote sauvage. Dans ce cas, les individus portant une délétion dans le gène choisi sont identifiés directement et les effets de la mutation pourront être évalués dans la descendance de ces individus.
Au sens de la présente invention, le terme gène inclut aussi bien les séquences codantes que des séquences non codantes.
Également au sens de la présente invention, les mutants par réarrangement génétique sont des mutants de délétion, d'inversion, d'insertion ou de translocation ; toutefois, les mutants de délétion sont les plus fréquents.
Afin d'assurer un spectre de délétions le plus large possible, les rayonnements gamma et neutrons rapides seront utilisés préférentiellement comme agents mutagènes. D'autres agents physiques, tels que les ions lourds, provoquant majoritairement des délétions pourront également être utilisées comme mutagènes.
Les avantages d'une collection de mutants induits par les rayonnements ionisants, dans les conditions exposées ci-dessus, sont les suivants :

- le type de mutagène utilisé assure une couverture complète du génome, accompagnée d'une grande diversité des allèles produits qui vont de petites délétions permettant d'éliminer une partie d'un gène ou de ses séquences régulatrices, jusqu'à de grandes délétions permettant d'éliminer des clusters de gènes ou des domaines chromosomiques. L'analyse fonctionnelle de domaines chromosomiques chez les plantes n'est envisageable que grâce à ce type de mutations ; de plus, les délétions rendent accessibles une analyse du rôle de l'ADN non-codant.

- le développement de cette banque ne fait pas appel à la transgenèse ; il en découle une facilité de mise en place et une rapidité de constitution. Une collection qui couvre plusieurs fois le génome peut être rassemblée en moins d'un an chez *Arabidopsis.* En comparaison, les banques de mutants insertionnels ne seront saturantes, de manière opérationnelles qu'à moyen terme (5 ans). Par ailleurs, les mutants radio-induits ne sont pas soumis aux contraintes de confinement des organismes génétiquement modifiés ; ils peuvent être cultivés en plein champ et dans les laboratoires non équipés pour les OGM. En outre, la technique selon la présente invention est directement utilisable dans des espèces cultivées pour lesquelles la transgenèse est encore difficile. A ce titre le riz représente une cible privilégiée.

Selon un mode de mise en oeuvre avantageux du procédé selon l'invention, préalablement à l'étape (b) la sélection de la dose d'irradiation x comprend :

- l'irradiation d'un échantillon de graines M1 à une dose comprise entre 0,05 Gy et 2 kGy ; les doses appliquées dépendent du type de mutagène et de l'espèce considérée. Par exemple, les neutrons rapides à dose identique sont 5 à 10 fois plus efficaces que les rayons gamma du $^{60}$Co.
- la germination desdites graines, dans un milieu convenable (solide ou liquide) ;
- l'extraction, de l'ADN à partir des plantes en lots regroupant au moins 50 plantes, et la récolte des graines M2 desdites plantes en lots correspondant aux lots d'ADN ;
- l'amplification desdits ADN, dans les conditions exposées à l'étape (e) et la détection des séquences amplifiées pour identifier la présence de mutations dans un gène et la répétition de ces étapes d'amplification et de détection pour plusieurs gènes, ci-après dénommés gènes témoins ;
- la détermination du nombre de mutations moyen *m* par plante, à partir du nombre de mutations obtenues pour les gènes témoins en utilisant les formules 1, 2 et 3 et
- optionnellement l'obtention d'une courbe dose/nombre de mutations *m* par plante.

Ainsi, il est possible de tracer une courbe dose/nombre de mutations *m* par plante, qui servira de référence pour le choix de la dose.

Selon un autre mode de mise en oeuvre avantageux du procédé selon l'invention, le nombre moyen de mutations par gène $y_x$ présentes dans la collection (calculé conformément aux étapes **(a)** et **(b)** dudit procédé) est avantageusement au moins égal à 2, ceci afin de disposer d'un nombre d'allèles suffisants pour chaque gène.

De manière avantageuse, la méthode selon l'invention, qui présente un certain nombre d'avantages par rapport à la transgenèse, est applicable à n'importe quelle plante, notamment, les espèces pour lesquelles la transgenèse est difficile ou pas encore possible :

- en particulier, la collection de mutants obtenue est suffisamment représentative, dans la mesure où elle contient effectivement au moins un mutant pour chaque gène du génome de la plante étudiée ; le nombre de mutants à obtenir, évalué conformément aux étapes **(a)** et **(b)** du procédé selon l'invention, dépend du nombre total de gènes dans la plante étudiée et du nombre de mutations par individu, qui dépend lui-même du mutagène et de la dose de mutagène utilisés.
- le type de mutagène utilisé (irradiation), permet un repérage aisé des mutants par PCR (délétions) et permet d'isoler les mutants repérés de la collection pour les étudier.

Le procédé ci-dessus est notamment applicable à *Arabidopsis* dont le Nombre de gènes estimé à 20 000 (Bouchez et Hofte, 1998) ; dans ce cas, il faut environ 60 000 mutations pour une probabilité de 0,95 d'avoir une mutation dans un gène donné.

Pour ce qui concerne les autres espèces, si l'espèce est hermaphrodite et auto-compatible, le procédé à mettre en oeuvre est le même que celui exposé ci-dessus, à l'exception de la dose d'irradiation qui dépend de la taille du génome, exprimée en nombre de gènes et de la fréquence de mutations induites pour un gène donné. Cette fréquence est évaluée selon le même protocole que celui décrit ci-dessus. Si l'espèce est auto-incompatible, les plantes de génération M1 sont croisées avec des plantes compatibles de la même espèce, pour produire les graines M2. Cette opération sera répétée pour obtenir les graines de générations suivantes.

La présente invention a également pour objet une collection de mutants, issus d'une plante A, caractérisée en ce que chaque mutant la constituant comprend au moins une mutation de délétion dans un gène du génome de ladite plante A, l'ensemble desdits mutants formant une population dont chaque élément comprend au moins une mutation pour un gène du génome de ladite plante A, ladite collection (graines ou ADN) représentant au moins une fois et de préférence 5 à 10 fois le génome.

Ladite collection est susceptible d'être obtenue à l'aide d'un procédé de production d'une collection de mutants tel que défini ci-dessus.

Ladite collection est constituée de graines de génération M2 et/ou de l'ADN extrait de ces graines, à partir d'une fraction représentative desdites graines.

Pour chaque famille M2, l'ADN est extrait d'une fraction représentative des graines de cette famille.

Outres les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1 illustre le principe de la détection de délétions par PCR suivi d'électrophorèse;
- la figure 2 illustre la recherche pyramidale de délétions dans la collection de mutants. Les amplifications du type décrit en figure 1 sont appliquées à des lots de taille décroissante jusqu'à identifier un individu portant la délétion recherchée.
- la figure 3 est un schéma illustrant l'utilisation des puces à oligonucléotides pour la détection et la localisation de délétions et de réarrangements chromosomiques chez des mutants issus de la collection. Ceci permettra d'une part d'accélérer le clonage de gènes affectés chez des mutants isolés sur la base d'un phénotype et d'autre part, d'envisager la localisation systématique des délétions chez les mutants de la collection. Ces informations seront consignées dans une base de données et la consultation de cette base autorisera un accès direct aux mutants dans un gène donné, sans passer par l'étape de criblage par PCR décrite ci-dessus.

Il doit être bien entendu, toutefois, que ces exemples sont donnés uniquement à titre d'illustration de l'objet de l'invention, dont ils ne constituent en aucune manière une limitation.

### <u>EXEMPLE 1</u> : Courbe dose/nombre de mutations induites par plante.

- l'irradiation d'un échantillon d'au moins 1000 graines sauvages à une dose comprise entre 0,05 Gy et 2 kGy ;
- la germination desdites graines sur un substrat approprié (terreau, par exemple) ;
- l'extraction de l'ADN des plantes issues de la germination des graines, par lots de 100, à partir soit des feuilles, soit des inflorescences selon le protocole suivant :

   1) Prélever les tissus frais, feuilles ou inflorescences et les congeler dans de l'azote liquide.
   2) Broyer les tissus congelés dans un broyeur planétaire (modèle PM400, marque RETSCH, Allemagne) et transférer le broyat dans des tubes de taille adéquate.
   3) Ajouter du tampon d'extraction à raison de 5 ml par g de tissus frais. Agiter pendant 15 min à 37°C.
   4) Ajouter un volume équivalent de mélange phénol-chloroforme-alcool isoamylique (25:24:1). Le phénol est tamponné par du Tris 50 mM, pH 8. Agiter pendant 10 min à 37°C.
   5) Centrifuger le mélange à 2 500 g pendant 5 min.
   6) Récupérer la phase aqueuse et la transférer dans un tube propre. Ajouter 1/10 du volume d'une solution 3 M d'acétate de sodium pH 5,2, puis 1 volume d'isopropanol. Agiter brièvement pour homogénéiser et centrifuger le tout à 10 000 g pendant 3 min.
   7) Eliminer le surnageant et reprendre le culot dans un volume de TE (tampon 10 mM Tris HCl pH 8,0 ; 5 mM EDTA). Ajouter 1/10 du volume d'une solution d'acétate de sodium 3 M pH 5,2. Puis 1 volume d'éthanol 100 %. Agiter et centrifuger pendant 5 min à 10 000 g.
   8) Eliminer le surnageant et reprendre le culot dans 1/10 du volume de tampon TE. La concentration obtenue varie entre 100 et 500 ng/$\mu$l.

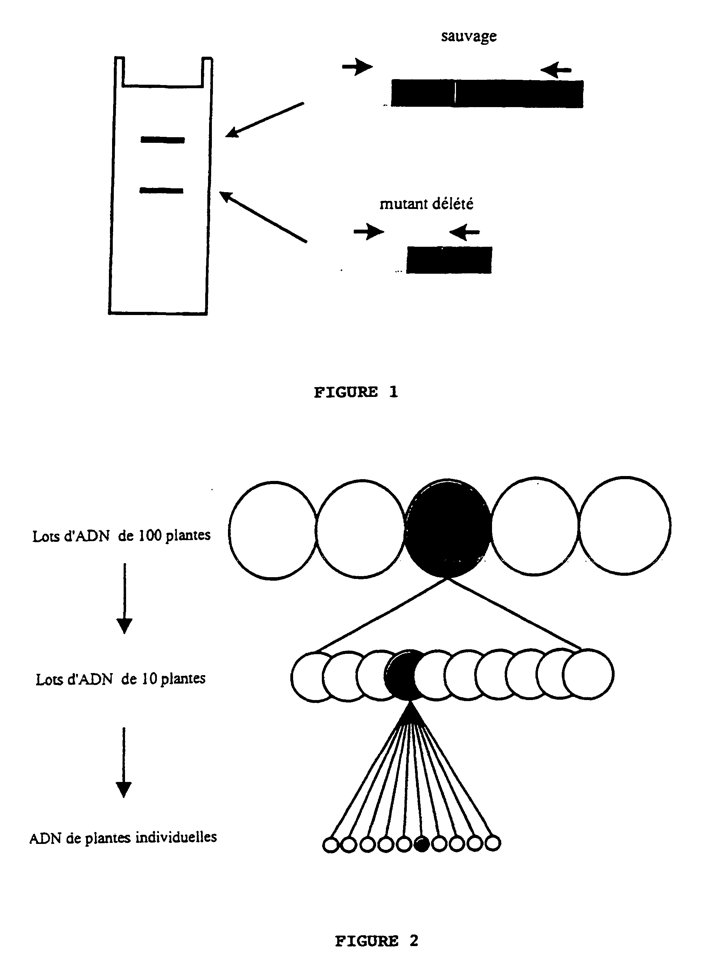

- l'amplification par PCR audit ADN, à l'aide d'amorces convenables, c'est-à-dire aptes à détecter des réarrangements génétiques, dans l'intervalle compris entre lesdites amorces, dans des conditions standards, suivie de la détection des séquences amplifiées par électrophorèse (figure 1) pour identifier la présence de mutations dans un gène et la répétition de cette étape d'amplification pour plusieurs gènes (1 à 10) et
- la détermination du nombre de mutations moyen m par plante, à partir du nombre de mutations obtenues pour les gènes témoins en utilisant les formules 1, 2 et 3 et
- optionnellement l'obtention d'une courbe dose/nombre de mutations *m* par plante.

Les conditions de PCR sont les suivantes : les réactions sont effectuées dans un volume de 25 à 50 $\mu$l contenant 1 à 2 unités de polymérase Taq, 10 mM Tris HCl pH 8,3, 50 mM KCl, 1,5 mM MgCl$_2$, 0,001 % gélatine, 0,2 mM de chaque nucléotide (dATP, dGTP, dCTP, dTTP), 0,2 $\mu$M de chaque amorce et de 10 à 20 ng d'ADN servant de matrice. Après une dénaturation initiale de 2 min à 94°C, suivent 30 cycles avec chacun une étape de dénaturation de 30 s à 94°C, une étape d'appariement des amorces de 30 s à une température variant de 45°C à 65°C selon les amorces, et une étape d'extension à 72°C pour une durée de 30 s à 4 min selon la taille du fragment à amplifier. Pour les fragments de grande taille (supérieure à 4 kb), la polymérase Taq est remplacée par un mélange de polymérase Pwo et de polymérase Taq dans un rapport de 1:100 en unités. Dans ce cas, la température d'élongation est de 68°C et la durée portée à 5 min ou plus si nécessaire.

**EXEMPLE 2 : Etablissement d'une banque chez _Arabidopsis thaliana_**

Pour _Arabidopsis thaliana,_ l'exemple 1 permet de sélectionner, comme dose d'irradiation _x_, 300 Gy. Par une source de $^{60}$Co (irradiateur CIGAL).

Afin d'assurer un spectre de délétions le plus large possible, les rayonnements gamma et neutrons rapides seront utilisés préférentiellement comme agents mutagènes. La taille de la banque (correspondant au nombre total de mutations **n**) doit être telle (voir formules 1, 2 et 3) que chaque région du génome soit mutée plusieurs fois. En effet, la caractérisation d'un phénotype mutant a d'autant plus de valeur qu'elle est confirmée par des allèles indépendants. Une collection représentant 5 à 10 fois le génome est donc à réaliser pour disposer d'une diversité d'allèles suffisante. Chez _Arabidopsis_ par exemple, le nombre total de gènes étant estimé à 20-25 000 et le nombre moyen de mutations dans une graine irradiée à 300 Gy (gamma) étant d'environ 100, la collection de mutants devra comporter 5-10 000 plantes.

Conformément au procédé selon l'invention, $p_n$=0,95 et $20\ 000 \leq N \geq 25\ 000$.

Les graines M2 sont récoltées pour chaque plante M1 individuellement (=M2i.gr). L'ADN est extrait selon le protocole décrit à l'Exemple 1, à partir de plantules M2 poussant en milieu liquide, à raison de 100 graines par plante M1 et en regroupant les graines issues de 10 plantes M1 (=10M2i.ADN).

Pour réduire le nombre de PCR à effectuer à l'étape (e) de criblage des réarrangement génétiques (délétions), les ADN des lots de graines M2 issues de 10 plantes M1 (=10M2i.ADN) sont eux même regroupés en super-lots de 10 (=100 M2i.ADN) (figure 2), en mélangeant en quantités égales les ADN des lots contribuant à un super-lot.

En outre, les graines M2 sont organisées en matrice 10x10 où chaque case de la matrice correspond aux graines M2 issues d'une plante M1 et les ADN sont extraits pour les deux dimensions (colonnes et lignes) de ces matrices. Ainsi les lots 10M2i.ADN correspondent à une colonne ou une ligne de ces matrices et les super-lots 100M2i. ADN correspondent à l'ensemole d'une matrice.

Ainsi, pour une collection faite à partir de 10 000 plantes M1, il y a 100 super-lots 100M2i.ADN. Il faudra donc effectuer 100 PCR (1 par super-lot), de manière à cribler la totalité de la banque. Sur les super-lots positifs, 2x 10 PCR correspondant aux 10 colonnes et 10 lignes des matrices, seront effectués pour identifier les graines M2i issues d'une plantes M1 portant une mutation détecté.

Pour identifier au moins une plante individuelle portant la mutation trouvée, il faut semer 50-100 graines M2i, extraire l'ADN des plantes individuellement et effectuer la PCR avec les mêmes amorces.

**EXEMPLE 3 : Cribles PCR sélectionnés pour _Arabidopsis thaliana._**

Un crible a été effectué sur un gène _d'Arabidopsis thaliana_ homologue du gène humain ATM _(Ataxia telangiectasia mutated);_ le gène est inclus dans la séquence du clone BAC T24C20 n° EMBL AL096856. La collection utilisée repose sur 5 000 lignées M1, obtenues dans les conditions décrites selon l'exemple 2. Les amorces utilisées ont les séquences suivantes :

ATM29 : 5'-GATTGATTAGATAGACAGAGC-3'

ATM40 : 5'AAAAAATTGGAAGCATATAGAAGGTTGGTTACA-3'

Les réactions d'amplification ont été réalisées dans les conditions décrites plus haut, avec le mélange de polymérases Taq et Pwo, à une température d'appariement des amorces de 55°C, un temps d'élongation de 5 min et sur 30 cycles. La taille du fragment amplifié à partir d'ADN génomique sauvage est de 5714 pb. Deux délétions de 2 300 et 1 600 pb respectivement ont pu être détectées dans deux lots différents, et les plantes portant ces délétions ont été isolées.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée,

## EP 1 223 798 B1

de la présente invention comme defini par les revendications.

**Références bibliographiques**

- Azpiroz-Leehan R et al. (1997) T-DNA insertion mutagenesis in *Arabidopsis :* going back and forth. Trends Genet. 13 : 152-156.
- Bouchez D et al. (1998) Functional genomics in plants. Plant Phys. 118 : 725-732.
- Bruggeman E et al. (1996) Analysis of fast neutron-generated mutants at the *Arabidopsis thaliana HY4* locus. The Plant Journal 10 : 755-760.
- Cattanach BM et al. (1993). Large deletions and other gross forms of chromosome imbalance compatible with viability and fertility in the mouse. Nat. Genet. 3 : 56-61.
- Cecchini E et al. (1998) Characterization of gamma irradiation-induced deletion mutants at a selectable locus in *Arabidopsis.* Mutat. Res. 401 : 199-206.
- Chakrabarti S et al. (1983) Frequency of gamma ray-induced spécifie locus and recessive lethal mutations in mature germ cells of the zebrafish, *Brachydanio rerio.* Genetics 103 : 109-123.
- Chiang P et al. (1999) A fluorescent quantitative PCR approach to map gene deletions in the Drosophila genome. Genetics 153 : 1313-1316.
- Fujii T (1978) Studies in neutron mutagenesis in maize. Effects of dose fractionation and cell moisture status on mutation induction. Radioisotopes 27 : 642-647.
- Fuscoe JC et al. (1992) Analysis of X-ray-induced HPRT mutations in CHO cells : Insertion and deletions. Mut. Res. 269 : 171-183.
- Fritz A et al. (1996) Identification of selected gamma-ray induced deficiencies in Zebrafish using multiplex polymerase chain reaction. Genetics 144 : 1735-1745.
- Giver CR et al. (1995) Mutational spectrum of X-ray induced TK-human cell mutants. Carcinogenesis 16 : 267-275.
- Jansen G et al. (1997) Reverse genetics by chemical mutagenesis in *Caenorhabditis elegans.* Nat. Genet. 17 : 119-121.
- Kaiser K et al. (1990) «Site-selected » transposon mutagenesis of *Drosophila.* Proc. Natl. Acad. Sci. USA 87 : 1686-1690.
- Kelley MR et al. (1985) Molecular analysis of X-ray-induced alcohol dehydrogenase (ADH) null mutations in *Drosophila melanogaster.* Genetics 109 : 365-377.
- Kubota Y et al. (1992) Detection of gamma ray-induced DNA damages in malformed dominant lethal embryos of the Japanese medaka *(Oryzia latipes)* using AP-PCR fingerprinting. Mutat. Res. 283 : 263-270.
- Martienssen RA (1998) Functional genomics : probing plant gene function and expression with transposons. Proc. Natl. Acad. Sci. USA 95 : 2021-2026.
- Muller HJ (1927) Artificial transmutation of the gene. Science 66 : 84-87.
- Purushothaman G (1969) Induced mutagenesis in rice. Madras Agr. J. 56 : 110-119.
- Redei GP(1974) *Arabidopsis* as a genetic tool. Annu. Rev. Genet. 9 : 111-127.
- Sagan M et al. (1995) Selection of nodulation and mycorrhizal mutants in the model plant Medicago truncatula (Gaertn.) after y-ray mutagenesis. Plant Sci. 111 : 63-71.
- Sankaranarayanan K. (1982) Genetic effects of ionizing radiation in multicellular eukaryotes and the assessment of genetic radiation hazards in Man, Elsevier, Amsterdam.
- Schwartz JF, (1991) Differential locus sensitivity to mutation induction by ionizing radiations of différent LETs in Chinesehamster ovary K1 cells. Carcinogenesis 12 : 1721-1726.
- Shirley BW et al. (1992) Effects of ionizing radiation on a plant genome : Analysis of two *Arabidopsis transparent testa* mutations. Plant Cell 4 : 333-347.
- Smith V et al. (1995) genetic footprinting : a genomic strategy for determining a gene's function given its séquence. Proc. Natl. Acad. Sci. USA 92 : 6479-6483.
- Sun TP et al. (1992) Cloning the *Arabidopsis GAI* locus by genomic substraction. Plant Cell 4 : 119-128.
- Turker MS et al. (1997) Molecular évidence for the induction of large interstitial deletions on mouse chromosome 8 by ionizing radiation. Mut. Res. 374 : 201-208.
- Zwaal RR et al. (1993) Target-selected gene inactivation in Caenorhabditis elegans by using a frozen transposon insertion mutant bank. Proc. Natl. Acad. Sci. USA 90 : 7431-7435.

**Revendications**

1. °) Procédé de production d'une collection ou banque de mutants pour l'étude de la fonction de chaque gène d'une plante A sélectionnée, **caractérisé en ce qu'**il comprend :

   **(a)** une estimation théorique du nombre **n** de mutations, induites par les rayonnements ionisants, nécessaires et suffisantes pour que chaque gène du génome de ladite plante A soit affectée par au moins l'une d'entre elles, A partir de la formule :

$$P_n = 1-((N-1)/N)^n \qquad \text{(formule 1)},$$

   dans laquelle :

   $p_n$ représente la probabilité qu'un gène donné soit muté et,
   N représente le nombre total estimé de gènes d'un organisme donné

   **(b)** l'irradiation de graines sauvages, par une dose d'irradiation $x$, choisie, de manière à obtenir une densité de mutation par plante $m$ et une fréquence de mutation dans un gène $y_x$ souhaitées, la relation entre x, $m$ et $y$ étant représentée par les formules 2 et 3 suivantes :

$$m=n/y_x \qquad \text{(formule 2)},$$

   dans laquelle **n** a la même signification qu'à la formule 1 et $m$ est le nombre de mutations par plante ;

$$y_x = ax + f_s \qquad \text{(formule 3)},$$

   dans laquelle:

   $\underline{a}$ est le facteur de mutagenèse et $f_s$ est la fréquence de mutation spontanée et
   l'obtention de graines, mutées de génération M1 mutées;

   **(c)** l'obtention des plantes de génération M1, issues des graines mutées M1 obtenues en **(b)**, par germination et culture ;
   **($d_1$)** la récolte des graines soeurs M2 pour chaque plante M1, l'extraction de l'ADN à partir de plantules soeurs M2 et le regroupement dudit ADN en lots correspondant à plusieurs familles M2 qui servent de matrice pour la recherche des réarrangements génétiques ; et
   **($e_1$)** la détection des familles M2 portant une mutation dans un gène donné, effectuée sur l'ADN obtenu en **($d_1$)**, par un criblage reposant sur des techniques de PCR aptes à détecter des réarrangements génétiques, réalisé sur des lots d'ADN de taille décroissante.

2. Procédé de production d'une collection ou banque de mutants pour l'étude de la fonction de chaque gène d'une plante A sélectionnée, **caractérisé en ce qu'**il comprend :

   **(a)** une estimation théorique du nombre **n** de mutations, induites par les rayonnements ionisants, nécessaires et suffisantes pour que chaque gène du génome de ladite plante A soit affecté par au moins l'une d'entre elles, à partir de la formule:

$$p_n = 1-((N-1)/N)^n \qquad \text{(formule 1)},$$

   dans laquelle

   $p_n$ représente la probabilité qu'un gène donné soit muté et,
   N représente le nombre total estimé de gènes d'un organisme donné ;

**(b)** l'irradiation de graines sauvages, par une dose d'irradiation $x$, choisie, de manière à obtenir une densité de mutation par plante $m$ et une fréquence de mutation dans un gène $y_x$ souhaitées, la relation entre $x$, $m$ et $y$, étant représentée par les formules 2 et 3 suivantes :

$$m = n/y_x \qquad \text{(formule 2),}$$

dans laquelle $n$ a la même signification qu'à la formule 1 et $m$ est le nombre de mutations par plante ;

$$y_x = ax + f_s \qquad \text{(formule 3),}$$

dans laquelle :

$\underline{a}$ est le facteur de mutagenèse et $f_s$ est la fréquence de mutation spontanée et l'obtention de graines mutées de génération M1 mutées ;

**(c)** l'obtention des plantes de génération M1, issues des graines mutées M1 obtenues en **(b)**, par germination et culture;

**(d₂)** la récolte des graines soeurs M2 pour chaque plante M1, l'obtention des plantes de génération M2 par germination et culture, et l'extraction de l'ADN de plantes M2 individuelles ; et

**(e₂)** la détection et la localisation des réarrangements portés par ces plantes soit par marquage et hybridation de chaque ADN obtenu en **(d₂)** avec une puce de génotypage à oligonucléotides, soit par PCR quantitative.

3.  Procédé selon la revendication 1 ou la revendication 2, **caractérisé en ce que**, préalablement à l'étape **(b)**, la sélection de la dose d'irradiation $x$, comprend :

    -   l'irradiation d'un échantillon de graines M1 à une dose comprise entre 0,05 Gy e t 2 kGy, les doses appliquées dépendant du type de mutagène et de l'espèce considérée ;
    -   la germination desdites graines en milieu convenable ;
    -   l'extraction de l'ADN à partir des plantes en lots regroupant au moins 50 plantes, et la récolte des graines M2 desdites plantes en lots correspondant aux lots d'ADN;
    -   l'amplification desdits ADN, dans les conditions exposées à l'étape **(e)** et la détection des séquences amplifiées pour identifier la présence de mutations dans un gène et la répétition de ces étapes d'amplification et de détection pour plusieurs gènes ;
    -   la détermination du nombre de mutations moyen $m$ par plante, à partir du nombre de mutations obtenues pour les gènes témoins en utilisant les formules 1, 2 et 3 et
    -   optionnellement l'obtention d'une courbe dose/nombre de mutations $m$ par plante.

4.  Procédé selon la revendication 1 ou la revendication 2, **caractérisé en ce que** le nombre moyen de mutations par gène $y_x$, présentes dans la collection, calculé conformément aux étapes **(a)** et **(b)** dudit procédé, est avantageusement au moins égal à 2.

5.  Procédé selon la revendication 1, **caractérisé en ce que** pour réduire le nombre de PCR à effectuer à l'étape **(e₁)** de criblage des réarrangements génétiques, les ADN des lots de graines M2 issues de plantes sont eux-mêmes regroupés en super-lots.

6.  Collection de mutants, issus d'une plante A, **caractérisée en ce qu'**elle est susceptible d'être obtenue à l'aide d'un procédé de production d'une collection de mutants selon l'une quelconque des revendications 1 à 5 et **en ce que** chaque mutant la constituant comprend au moins une mutation de délétion dans un gène du génome de ladite plante A, l'ensemble desdits mutants formant une population dont chaque élément comprend au moins une mutation pour un gène du génome de ladite plante A, ladite collection représentant au moins une fois et de préférence 5 à 10 fois le génome, ladite collection étant constituée de graines de génération M2 et/ou de l'ADN extrait de ces graines, à partir d'une fraction représentative desdites graines.

**Patentansprüche**

1. Verfahren zum Erzeugen einer Sammlung oder Mutantenbank zum Studium der Funktion eines jeden Gens einer ausgewählten Pflanze A, **dadurch gekennzeichnet, daß** das Verfahren folgende Schritte umfaßt:

(a) theoretisches Abschätzen der Anzahl **n** der durch ionosierende Strahlung induzierten Mutationen, welche notwendig und ausreichend ist für jedes Gen der genannten Pflanze A, wobei wenigstens eine unter ihnen betroffen ist, ausgehend von der Formel :

$$p_n = 1 - ((N-1)/N)^n \qquad \text{(Formel 1),}$$

worin:

$p_n$ die Wahrscheinlichkeit wiedergibt, daß ein bestimmtes Gen mutiert ist, und
N die geschätzte Gesamtzahl der Gene eines bestimmten Organismus wiedergibt,

(b) Bestrahlen der wilden Körner mit einer Strahlendosis x, welche so gewählt ist, daß eine gewünschte Mutationsdichte pro Pflanze *m* und eine Mutationsfrequenz in einem Gen $y_x$ erhalten wird, wobei das Verhältnis zwischen x, *m* und y durch die nachfolgenden Formeln 2 und 3 wiedergegeben wird:

$$m = n/y_x \qquad \text{(Formel 2),}$$

worin **n** dieselbe Bedeutung besitzt wie in Formel 1 und *m* die Zahl der Mutationen pro Pflanze ist,

$$y_x = ax + f_s \qquad \text{(Formel 3),}$$

worin:

$\underline{a}$ der Mutagenesefaktor ist und $f_s$ die Frequenz der spontanen Mutation und der erhaltenen mutierten Körner der mutierten Generation M1;

(c) Gewinnen von Pflanzen der Generation M1, die aus mutierten Körnern der Generation M1 stammen, durch Keimen und Kultivieren;
($d_1$) Ernten der Schwesterkörner M2 für jede Pflanze M1, Extrahieren der DNA, ausgehend von den Schwesterkeimlingen M2 und Umgruppieren dieser DNA in Chargen entsprechend mehreren Familien M2, die als Matrix zur Suche von genetischen Neuordnungen dienen; und
($e_1$) Detektieren von Familien M2, welche eine Mutation in einem bestimmten Gen tragen, die auf der in ($d_1$) durch ein auf PCR-Techniken beruhendes Screening erhaltenen DNA bewirkt wurde, wobei die PCR-Techniken zur Detektion von genetischen Neuordnungen geeignet sind, welche in Chargen von in der Grösse abnehmender DNA realisiert sind.

2. Verfahren zum Erzeugen einer Sammlung oder Mutantenbank zum Studium der Funktion eines jeden Gens einer ausgewählten Pflanze A, **dadurch gekennzeichnet, daß** das Verfahren folgende Schritte umfaßt:

(a) theoretisches Abschätzen der Anzahl n der durch ionisierende Strahlung induzierten Mutationen, welche notwendig und ausreichend ist für jedes Gen der genannten Pflanze A, wobei wenigstens eine unter ihnen betroffen ist, ausgehend von der Formel :

$$P_n = 1 - ((N-1)/N)^n \qquad \text{(Formel 1),}$$

worin:

$P_n$ die Wahrscheinlichkeit wiedergibt, daß ein bestimmtes Gen mutiert ist, und

N die geschätzte Gesamtzahl der Gene eines bestimmten Organismus wiedergibt,

(b) Bestrahlen der wilden Kömer mit einer Strahlendosis x, welche so gewählt ist, daß eine gewünschte Mutationsdichte pro Pflanze $m$ und eine Mutationsfrequenz in einem Gen $y_x$ erhalten wird, wobei das Verhältnis zwischen x, $m$ und y durch die nachfolgenden Formeln 2 und 3 wiedergegeben wird:

$$m=n/y_x \qquad \text{(Formel 2)},$$

worin **n** dieselbe Bedeutung besitzt wie in Formel 1 und $m$ die Zahl der Mutationen pro Pflanze ist,

$$y_x=ax+f_s \qquad \text{(Formel 3)},$$

worin:

$\underline{a}$ der Mutagenesefaktor ist und $f_s$ die Frequenz der spontanen Mutation und der erhaltenen mutierten Körner der mutierten Generation M1;

(c) Beschaffen von Pflanzen der Generation M1, die aus mutierten Körnern der Generation M1 stammen, durch Keimen und Kultivieren;

($d_2$) Ernten der Schwesterkörner M2 für jede Pflanze M1, Gewinnen von Pflanzen der Generation M2 durch Keimen und Kultivieren und Extraktion der DNA der einzelnen Pflanzen M2; und

($e_2$) Detektieren und Lokalisieren der von den Pflanzen getragenen Neuordnungen, sei es mittels Markieren und Hybridisieren von jeder in ($d_2$) erhaltenen DNA mit einem Oligonucleotid-Chip zur Bestimmung des Genotyps, sei es mittels quantitativer PCR.

3. Verfahren gemäß Anspruch 1 oder Anspruch 2, **dadurch gekennzeichnet, daß** vor der Stufe (b) die Auswahl der Strahlungsdosis x umfaßt:

- Bestrahlen einer Probe von Körnern M1 mit einer Dosis zwischen 0,05 Gy und 2 kGy, wobei die angewendete Dosis vom Typ des Mutagens und der betrachteten Spezies abhängt;
- Keimen der genannten Körner in einem geeigneten Milieu;
- Extrahieren der DNA ausgehend von Pflanzen, die in Chargen von mindestens 50 Pflanzen zusammengefaßt sind, und Ernten der Körner M2 der genannten Pflanzen in Chargen, die den Chargen der DNA entsprechen;
- Amplifikation der genannten DNA unter den in Stufe (e) dargelegten Bedingungen und Detektieren der amplifizierten Sequenzen zum Identifizieren des Vorliegens von Mutationen in einem Gen und Wiederholen der Stufen der Amplifikation und des Detektierens für mehrere Gene;
- Bestimmen der mittleren Zahl der Mutationen $m$ pro Pflanze, ausgehend von der Zahl der erhaltenen Mutationen für die nachgewiesenen Gene unter Verwendung der Formeln 1, 2 und 3 und
- optional Gewinnen einer Kurve Dosis/Zahl der Mutationen $m$ pro Pflanze.

4. Verfahren gemäß Anspruch 1 oder Anspruch 2, **dadurch gekennzeichnet, daß** die in Übereinstimmung mit den Stufen (a) und (b) des genannten Verfahrens berechnete mittlere Zahl der Mutationen pro Gen $y_x$, die in der Sammlung vorliegen, vorteilhafterweise wenigstens gleich 2 ist.

5. Verfahren gemäß Anspruch 1, **dadurch gekennzeichnet, daß** zur Verminderung der Zahl der durchzuführenden PCR in der Stufe ($e_1$) des Screenings der genetischen Neuordnungen die DNA der von den Pflanzen stammenden Körner M2 selbst in Super-Chargen zusammengefaßt sind.

6. Sammlung von Mutanten, die von einer Pflanze A stammen, **dadurch gekennzeichnet, daß** sie mit Hilfe eines Verfahrens zur Herstellung einer Sammlung von Mutanten gemäß einem der Ansprüche 1 bis 5 erhältlich ist und daß jede der die Sammlung bildenden Mutanten wenigstens eine Deletionsmutation in einem Gen des Genoms der genannten Pflanze A umfaßt, wobei das Ensemble der genannten Mutanten eine Population bildet, von der jedes Element wenigstens eine Mutation für ein Gen des Genoms der genannten Pflanze A umfaßt, und die genannte Sammlung das Genom wenigstens ein Mal und bevorzugt 5 bis 10 Mal repräsentiert und die genannte Sammlung aus Körnern der Generation M2 und/oder von einem DNA-Extrakt dieser Körner, ausgehend von einer repräsentativen Fraktion der genannten Körner, gebildet ist.

**Claims**

1. Process for the production of a group or bank of mutants to study the function of each gene of a chosen plant A, **characterized in that** it comprises:

    (a) a theoretical estimate of the number n of mutations, induced by ionising radiation, necessary and sufficient to ensure that each gene of the genome of the said plant A will be affected by at least one of them, based on the equation:

$$p_n = 1 - ((N - 1)/N)^n \qquad \text{(Equation 1)}$$

    in which:

    $p_n$ represents the probability that a given gene will be mutated and
    N represents the total estimated number of genes of a given organism;

    (b) irradiation of wild seeds by an irradiation dose *x* chosen in such a way as to obtain a required mutation density per plant *m* and a required mutation frequency in a gene $y_x$, the relationship between *x, m and y* being represented by the following Equations 2 and 3:

$$m = n/ y_x \qquad \text{(Equation 2),}$$

    in which **n** has the same meaning as in Equation 1 and *m* is the number of mutations per plant;

$$y_x = ax + f_s \qquad \text{(Equation 3),}$$

    in which:

    $\underline{a}$ is the mutagenesis factor and $f_s$ is the frequency of spontaneous mutation and the obtaining of mutated seeds of the M1 mutated generation;

    (c) obtaining plants of the M1 generation originating from mutated seeds M1 obtained in (b) by germination and cultivation;

    ($d_1$) harvesting sister seeds M2 for each M1 plant, extracting the DNA from the M2 sister plantlets and collecting together the said DNA into batches corresponding to several M2 families that act as a matrix for research into genetic rearrangements; and

    ($e_1$) detection of M2 families carrying a mutation in a given gene, performed on the DNA obtained in ($d_1$), by screening based on PCR techniques that are capable of detecting genetic rearrangements, performed on batches of DNA of decreasing size.

2. Process for the production of a group or bank of mutants to study the function of each gene of a chosen plant A, **characterized in that** it comprises:

    (a) a theoretical estimate of the number **n** of mutations, induced by ionising radiation, necessary and sufficient to ensure that each gene of the genome of the said plant A will be affected by at least one of them, based on the equation:

$$p_n = 1 - ((N - 1)/N)^n \qquad \text{(Equation 1)}$$

in which:

$p_n$ represents the probability that a given gene will be mutated and
N represents the total estimated number of genes of a given organism;

(b) irradiation of wild seeds by an irradiation dose $x$ chosen in such a way as to obtain a required mutation density per plant $m$ and a required mutation frequency in a gene $y_x$, the relationship between $x, m$ and $y$ being represented by the following Equations 2 and 3:

$$m = n/\, y_x \qquad\qquad \text{(Equation 2)},$$

in which $n$ has the same meaning as in Equation 1 and $m$ is the number of mutations per plant;

$$y_x = ax + f_s \qquad\qquad \text{(Equation 3)},$$

in which:

$\underline{a}$ is the mutagenesis factor and $f_s$ is the frequency of spontaneous mutation and the obtaining of mutated seeds of the M1 mutated generation;

(c) obtaining plants of the M1 generation originating from mutated seeds M1 obtained in (b) by germination and cultivation;

($d_2$) harvesting sister seeds M2 for each M1 plant, obtaining plants of the M2 generation by germination and cultivation, and extracting the DNA of individual M2 plants; and

($e_2$) the detecting and localisation of rearrangements carried by these plants either by labelling and hybridisation of each DNA obtained in ($d_2$) with an oligonucleotides genotyping chip or by quantitative PCR.

3. Process according to Claim 1 or Claim 2, **characterized in that**, prior to stage (b), the selection of the irradiation dose $x$ comprises:

- irradiation of a sample of M1 seeds at a dose comprised between 0.05 grays and 2 kilograys, the doses administered depending on the type of mutagenesis and on the species in question;

- germination of the said seeds in a suitable medium;

- extraction of the DNA from plants in batches that are a group of at least 50 plants, and harvesting the M2 seeds of the said plants in batches corresponding to the DNA batches;

- amplification of the said DNA under the conditions described in stage (e) and the detection of amplified sequences to identify the presence of mutations in a gene, and the repetition of these stages of amplification and detection for several genes;

- the determination of the average number of mutations $m$ per plant, based on the number of mutations obtained for the control genes, by using Equations 1, 2 and 3, and

- optionally obtaining a curve of the dose/number of mutations $m$ per plant.

4. Process according to Claim 1 or Claim 2, **characterized in that** the average number of mutations per gene $y_x$ present in the group, calculated according to stages (a) and (b) of the said process, is advantageously at least equal to 2.

5. Process according to Claim I, **characterized in that**, in order to reduce the number of PCR to be performed at stage ($e_1$) of the genetic rearrangements screening, the DNA from the batches of M2 seeds originating from the plants are themselves collected into super-batches.

6. Group of mutants originating from a plant A, **characterized in that** it is obtainable using a process for producing a group of mutants according to any one of the Claims 1 to 5, and **in that** each mutant constituting it comprises at least one deletion mutation in a gene of the genome of the said plant A, the entirety of the said mutants forming a population of which each element comprises at least one mutation for a gene of the genome of the said plant A, the said group representing the genome at least once and preferably 5 to 10 times, the said group consisting of seeds of the M2 generation and/or of the DNA extracted from these seeds, based on a representative fraction of the said seeds.

sauvage

mutant délété

**FIGURE 1**

Lots d'ADN de 100 plantes

Lots d'ADN de 10 plantes

ADN de plantes individuelles

**FIGURE 2**

Localisation rapide de délétions à l'aide des puces à oligos

SAUVAGE

MUTANT

FIGURE 3

EP 1 223 798 B1